Europäisches Patentamt

European Patent Office

Office européen des brevets

(19)

(11) Veröffentlichungsnummer: 0 133 537
A2

## (12) EUROPÄISCHE PATENTANMELDUNG

(21) Anmeldenummer: 84109068.1

(22) Anmeldetag: 01.08.84

(51) Int. Cl.⁴: **C 08 G 18/48**
C 08 G 18/78, C 08 G 18/79
E 04 C 2/20, A 61 L 17/00

(30) Priorität: 13.08.83 DE 3329392

(43) Veröffentlichungstag der Anmeldung:
27.02.85 Patentblatt 85/9

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Wegner, Christian, Dr.
Roggendorfstrasse 67
D-5000 Köln 80(DE)

(54) Thermisch verformbares Konstruktionsmaterial, Verfahren zu dessen Herstellung und dessen Verwendung.

(57) Konstruktionsmaterial auf Basis von Polyurethan, dadurch gekennzeichnet, daß es erhältlich ist durch Umsetzung von
(a) mindestens einem araliphatischen und/oder cycloaliphatischen Diisocyanat mit
(b) mindestens einem vorwiegend aus Propylenoxideinheiten aufgebauten Polyether mit einem Molekulargewicht von 350 - 1.000, vorzugsweise 400 - 800, und einer OH-Zahl von 250 - 600, vorzugsweise 350 - 450,
wobei das NCO/OH-Verhältnis zwischen 0,75 : 1 und 1,25 : 1, vorzugsweise zwischen 0,9 : 1 und 1,1 : 1, liegt.

EP 0 133 537 A2

BAYER AKTIENGESELLSCHAFT          5090 Leverkusen, Bayerwerk

Konzernverwaltung RP
Patentabteilung

Thermisch verformbares Konstruktionsmaterial,
Verfahren zu dessen Herstellung und dessen Verwendung

Die vorliegende Erfindung betrifft ein Konstruktionsmaterial, welches bei Temperaturen bis ca. 45°C hart und schlagzäh ist, sich jedoch oberhalb 60°C manuell leicht verformen läßt, Verfahren zu dessen Herstellung, sowie seine Verwendung insbesondere für orthopädische Zwecke.

Die Verwendung thermisch verformbarer Materialien in der medizinischen Technik, insbesondere in der Orthopädie, ist bekannt.

So wird z.B. in der deutschen Auslegeschrift 2758216 eine auf ein flexibles Trägermaterial aufgebrachte Masse beschrieben, die nach Tauchen in warmes Wasser verformbar ist und sich bei Abkühlung verfestigt. Dieses Material wird bevorzugt für die Herstellung von Stützverbänden eingesetzt, indem es im erwärmten Zustand nach Art einer Gipsbinde appliziert wird. Bei Raumtemperatur ist das Material jedoch nicht hart wie ein schlagzäher Kunststoff, sondern es weist mehr hartgummiartige Konsistenz auf. Für

Le A 22 537 - Ausland

die Herstellung eines Stützverbandes aus diesem Material ist es daher notwendig, mehrere Lagen zu wickeln, um eine ausreichende Steifigkeit des Produkts zu erreichen. Für andere Anwendungen wie Schienen, Stützen und Schuheinlagen weist das Material nicht die geforderten mechanischen Eigenschaften (insbesondere die Steifigkeit) auf, nachteilig ist darüber hinaus seine fehlende Transparenz.

Für die Herstellung von Stützverbänden, bei denen Schienen eingesetzt werden, lehrt die deutsche Auslegeschrift 2618613 die Verwendung von thermisch verformbaren Hartschäumen. Nachteilig bei diesem Material ist, daß seine Weichmachung ca. 120°C warmen Dampf erfordert, wozu eine spezielle Apparatur vonnöten ist. Zur Herstellung von steifen, dünnwandigen Konstruktionen sind diese Schäume ebenfalls nicht geeignet, da sie nicht nur eine rauhe, porige Oberfläche aufweisen, sondern auch bei Raumtemperatur noch geringfügig flexibel sind.

Thermisch verformbare, harte, schlagzähe, durchsichtige Materialien sind jedoch beispielsweise durch Homo- oder Copolymerisation von Methacrylaten oder Acrylaten, sowie gegebenenfalls weiteren Vinyleinheiten aufweisenden Monomeren, wie z.B. Acrylnitril, herstellbar. Solche Werkstoffe werden vornehmlich in der Orthopädie eingesetzt. Nachteilig bei dieser Substanzklasse sind die zu einer Flexibilisierung notwendigen Temperaturen $> 140°C$. Dies erfordert wiederum spezielle Apparaturen für die Erwärmung und Verformung.

Le A 22 537

Der vorliegenden Erfindung lag daher die Aufgabe zugrunde, ein Material bereitzustellen, welches bei Raumtemperatur hart, schlagzäh, formstabil und gegebenenfalls transparent, bei Temperaturen oberhalb von ca. 60°C jedoch flexibel, form- und anformbar ist.

Gegenstand der vorliegenden Erfindung ist daher ein bei Temperaturen < 45°C hartes, schlagzähes, formstabiles, gegebenenfalls transparentes, bei Temperaturen von > 60°C jedoch flexibles, form- und anformbares Konstruktionsmaterial aus Polyurethan, welches dadurch gekennzeichnet ist, daß es im wesentlichen erhältlich ist durch Reaktion von

(a) mindestens einem araliphatischen und/oder cycloaliphatischen Diisocyanat mit

(b) mindestens einem vorwiegend aus Propylenoxideinheiten aufgebauten Polyether mit einem Molekulargewicht von 350 - 1.000 und einer OH-Zahl von 250 - 600, wobei das NCO/OH-Verhältnis zwischen 0,75 : 1 und 1,25 : 1 liegt.

Gegenstand der vorliegenden Erfindung ist weiterhin die Verwendung des erfindungsgemäßen Konstruktionsmaterials als Abform- und manuell modellierbares Stützmaterial, insbesondere im medizinischen Bereich.

Die als Komponente (a) verwendeten Diisocyanate sind bei Raumtemperatur flüssige und lichtbeständige Verbindungen, die mindestens einen cycloaliphatischen oder aromatischen Kern aufweisen, deren Isocyanatgruppen jedoch nicht an aromatischen Kohlenstoff gebunden sind.

Le A 22 537

Beispiele solcher Diisocyanate sind das Isophorondiisocyanat, das Cyclohexan-1,4-diisocyanat, das 1-Methyl-
2,4-diisocyanatocyclohexan, das 4,4'-Dicyclohexylmethandiisocyanat und das Xylylendiisocyanat. Erfindungsgemäß
bevorzugt sind das Isophorondiisocyanat und das 4,4'-
Dicyclohexylmethandiisocyanat. Selbstverständlich können
erfindungsgemäß auch Diisocyanate der genannten Art eingesetzt werden, die in an sich bekannter Art modifiziert
(biuretisiert, trimerisiert, allophanatisiert, carbodiimidisiert, etc.) worden sind.

Bei den als Komponente (b) verwendeten Verbindungen handelt es sich um vorwiegend aus Propylenoxideinheiten aufgebaute Polyether mit einem Molekulargewicht von 350 bis
1.000, vorzugsweise 400 bis 800, und einer OH-Zahl von
250 bis 600, vorzugsweise 350 bis 450. Neben Propylenoxideinheiten können die Polyether (b) bis zu 40 Mol-%,
vorzugsweise weniger als 20 Mol-%, besonders bevorzugt
weniger als 10 Mol-% an sich bekannter anderer Polyethersequenzen (bevorzugt Ethylenoxideinheiten) enthalten.
Durch geeignete Variation der OH-Gruppen aufweisenden
Komponente lassen sich die Eigenschaften der erfindungsgemäßen Materialien in gewisser Weise steuern. Polyether
mit niederen Molekulargewichten und hohen OH-Zahlen
bringen z.B. eine höhere Vernetzungsdichte mit sich. Dadurch wird der Temperaturbereich der Wärmeverformung
heraufgesetzt. Erfindungsgemäß bevorzugt sind auf Trimethylolpropan, Pentaerythrit, Sorbit, Propylenglykol,
Glycerin oder Gemischen hiervon gestartete Propylenoxidpolyether. Selbstverständlich können auch Gemische von
verschiedenen Polyethern eingesetzt werden, sofern sie

Le A 22 537

im Mittel die o.a. Kenndaten aufweisen.

Teilweise (bis zu 50 Gew.-%, vorzugsweise weniger als 30 Gew.-%, besonders bevorzugt bis zu 20 Gew.-%) können die genannten Polyether auch durch andere OH-Gruppen aufweisende Komponenten ersetzt werden. Hier seien insbesondere genannt Tetrahydrofuranpolymerisate, Polycaprolactonpolyole sowie OH-funktionelle Polyester, insbesondere wenn sie Phthal- oder Adipinsäure-Einheiten aufweisen. Auch in diesem Fall hat das Gemisch aller OH-Gruppen aufweisenden Verbindungen ein mittleres Molekulargewicht von 350 bis 1.000 und eine mittlere OH-Zahl von 250 bis 600. Bei der Herstellung der erfindungsgemäßen Konstruktionsmaterialien beträgt das Mol-Verhältnis aller NCOzu OH-Gruppen 0,75 - 1,25 : 1, bevorzugt 0,9 - 1,1 : 1, speziell ca. 1 : 1.

Die erfindungsgemäßen Materialien sind bei Raumtemperatur klar, durchsichtig und schlagfest. Sie vergilben nicht unter Einfluß von Licht und es treten auch bei längerem Kontakt mit kaltem oder heißem Wasser keine durch Feuchtigkeitsaufnahme bedingten Trübungen auf. Bei Temperaturen $> 60°C$ werden die Materialien gummiartig weich und lassen sich beliebigen Verformungen unterwerfen, wie z.B. biegen, rollen oder falten. Überraschend und für die Handhabung dieser Materialien von Bedeutung ist jedoch die Tatsache, daß sich die Materialien im erwärmten, d.h. formbaren Zustand wie ein textiler Stoff oder ein Filzmaterial mit einer gewöhnlichen Schere oder anderen einfachen Schneidwerkzeugen unter Ausbildung sauberer Kanten schneiden lassen. In erkaltetem Zustand ist das Material hingegen auch den üblichen Bearbeitungsmethoden zugänglich, wie z.B. Bohren, Drehen, Schleifen oder Fräsen.

Le A 22 537

Die Herstellung der erfindungsgemäßen Materialien erfolgt nach den in der Polyurethantechnik üblichen Methoden. So ist es z.B. möglich, die Komponenten homogen zu vermischen, in eine Form zu bringen und bei erhöhter Temperatur auszuhärten. Die Aushärtung erfolgt bevorzugt in Anwesenheit an sich bekannter Katalysatoren, wobei Zinnorganische Verbindungen, wie z.B. Zinnoctoat oder Dibutylzinndilaurat besonders geeignet sind.

Die beschriebene Methode kann auch in vielerlei Hinsicht variiert werden. So können anstelle der monomeren Ausgangskomponenten (a) und (b), d. h. Diisocyanatgemisch und Polyol bzw. Polyolgemisch auch aus diesen Komponenten gebildete Präpolymere mit freien endständigen OH- bzw. NCO-Gruppen eingesetzt werden. Die NCO-Gruppen der eingesetzten Diisocyanate können anteilmäßig auch anderen, in der Isocyanatchemie bekannten Modifizierungsreaktionen unterworfen werden, wie z.B. Biuretisierung, Trimerisierung, Carbodiimidisierung, Harnstoffbildung und Allophanatisierung. Eine kontinuierliche Fertigung von Platten oder Stäben ist ebenfalls möglich, wenn mit einer Dosier- und Mischapparatur gearbeitet wird, an welche ein geheiztes Band angeschlossen ist, auf dem die Masse aushärtet.

Wie andere Kunststoffe können die erfindungsgemäßen Materialien auch in an sich bekannter Weise eingefärbt, lackiert oder mit anorganischen oder organischen Füllstoffen versehen werden.

Le A 22 537

Neben dem medizinischen Bereich, insbesondere der Orthopädie, bietet sich der Einsatz der erfindungsgemäßen Materialien immer dort an, wo in einfacher Weise verformbare und schneidbare Materialien mit guten mechanischen Eigenschaften benötigt werden, wie z.B. als Modellbaumaterial, Befestigungselement, Dekorationsmaterial, transparente Abdeckung, als Abformmaterial oder als Kunstglasmaterial für Verscheibungen.

Die nachstehend aufgeführten Beispiele erläutern die Erfindung. Mengenangaben sind als Gewichtsteile bzw. Gewichtsprozente zu verstehen.

Le A 22 537

Beispiel 1

100 Teile eines auf Trimethylolpropan gestarteten Propylenoxidpolyethers (OH-Zahl 375, MG 450) werden mit 73 Teilen Isophorondiisocyanat und 0,01 % Zinn-II-octoat homogen vermischt, entgast und bei 80°C zu einer Platte von 4 mm Dicke ausgehärtet. Man erhält ein transparentes, optisch einwandfreies, farbloses, schlagzähes Material, welches sich bei 65°C manuell leicht verformen bzw. schneiden läßt.

Beispiel 2

Man verfährt wie in Beispiel 1 angegeben, setzt jedoch als Isocyanatkomponente 86,5 g 4,4'-Dicyclohexylmethandiisocyanat ein. Man erhält ein Material wie in Beispiel 1, welches sich manuell ab 75°C verformen bzw. schneiden läßt.

Beispiel 3

63 Teile eines auf Sorbit und Propylenglykol gestarteten Propylenoxidpolyethers (OH-Zahl 480, MG 500) und 37 Teile eines auf Propylenglykol gestarteten Propylenoxidpolyethers (OH-Zahl 112, MG 1000) werden mit 68 Teilen Isophorondiisocyanat vermischt und wie in Beispiel 1 angegeben, ausgehärtet. Man erhält ein Material analog wie in Beispiel 1, welches sich jedoch erst ab 80°C manuell verformen bzw. schneiden läßt.

Le A 22 537

Beispiel 4

75 Teile eines auf Sorbit und Propylenglykol gestarteten Propylenoxidpolyethers (OH-Zahl 480, MG 500) und 25 Teile eines auf Propylenglykol gestarteten Propylenoxidpolyethers (OH-Zahl 112, MG 1000) werden mit 76,9 Teilen Isophorondiisocyanat vermischt und wie in Beispiel 1 angegeben, ausgehärtet. Man erhält ein Material analog wie in Beispiel 1, welches sich jedoch erst ab 90°C manuell verformen bzw. schneiden läßt.

Beispiel 5

50 Teile des in Beispiel 1 verwendeten Propylenoxidpolyethers, 20 Teile eines aus Phtalsäureanhydrid und Ethylenglykol aufgebauten Polyesters (OH-Zahl 280), 47,7 Teile Isophorondiisocyanat und 0,6 Teile Dibutylzinndilaurat werden homogen vermischt und wie in Beispiel 1 angegeben ausgehärtet. Man erhält ein Material analog Beispiel 1, welches sich ab 85°C leicht verformen läßt.

Le A 22 537

Patentansprüche

1. Konstruktionsmaterial auf Basis von Polyurethan, dadurch gekennzeichnet, daß es erhältlich ist durch Umsetzung von

   (a) mindestens einem araliphatischen und/oder cycloaliphatischen Diisocyanat mit

   (b) mindestens einem vorwiegend aus Propylenoxideinheiten aufgebauten Polyether mit einem Molekulargewicht von 350 - 1.000, vorzugsweise 400 - 800, und einer OH-Zahl von 250 - 600, vorzugsweise 350 - 450,

   wobei das NCO/OH-Verhältnis zwischen 0,75 : 1 und 1,25 : 1, vorzugsweise zwischen 0,9 : 1 und 1,1 : 1, liegt.

2. Konstruktionsmaterial nach Anspruch 1, dadurch gekennzeichnet, daß Komponente (a) teilweise biuretisiert, trimerisiert oder carbodiimidisiert ist.

3. Konstruktionsmaterial nach Anspruch 1 oder 2, dadurch gekennzeichnet, daß Komponente (a) Isophorondiisocyanat, 4,4'-Dicyclohexandiisocyanat oder Xylylendiisocyanat ist.

4. Konstruktionsmaterial nach Ansprüchen 1 bis 3, dadurch gekennzeichnet, daß Komponente (b) ein Polyether mit mindestens 60 Mol-%, vorzugsweise mindestens 80 Mol-%, Propylenoxideinheiten ist.

Le A 22 537

5. Konstruktionsmaterial nach Anspruch 4, dadurch gekennzeichnet, daß Komponente (b) auch Ethylenoxideinheiten enthält.

6. Konstruktionsmaterial nach Ansprüchen 1 bis 5, dadurch gekennzeichnet, daß Komponente (b) ein auf Propylenglykol, Glycerin, Trimethylolpropan, Pentaerythrit, Sorbit oder Gemischen hiervon gestarteter Polyether ist.

7. Konstruktionsmaterial nach Ansprüchen 1 bis 6, dadurch gekennzeichnet, daß Komponente (b) ein Gemisch verschiedener Polyether ist, welches ein mittleres Molekulargewicht von 350 - 1.000, vorzugsweise 400 - 800, und eine mittlere OH-Zahl von 250 - 600, vorzugsweise 350 - 450, aufweist.

8. Konstruktionsmaterial nach Ansprüchen 1 bis 7, dadurch gekennzeichnet, daß Komponente (b) bis zu 50 Gew.-%, vorzugsweise bis zu 30 Gew.-%, an anderen Hydroxylgruppen aufweisenden Verbindungen enthält, wobei die Summe aller Hydroxylgruppen aufweisenden Substanzen ein mittleres Molekulargewicht von 350 bis 1.000, vorzugsweise 400 - 800, und eine mittlere OH-Zahl von 250 - 600, vorzugsweise 350 - 450 , aufweist.

9. Verfahren zur Herstellung eines Konstruktionsmaterials nach Anspruch 1 bis 8, dadurch gekennzeichnet, daß man

Le A 22 537

(a) mindestens ein araliphatisches und/oder cycloaliphatisches Diisocyanat mit

(b) mindestens einem vorwiegend aus Propylenoxideinheiten aufgebauten Polyether sowie gegebenenfalls
bis zu 50 Gew.-%, bezogen auf Komponente (b),
an anderen Hydroxylgruppen aufweisenden Verbindungen, wobei die Summe aller eingesetzten Hydroxylgruppen aufweisenden Substanzen ein mittleres Molekulargewicht von 350 - 1.000, vorzugsweise 400 - 800, und eine mittlere OH-Zahl von
250 - 600, vorzugsweise 350 - 450, aufweist,

gegebenenfalls in mehreren Stufen, umsetzt, wobei das
NCO/OH-Verhältnis zwischen 0,75 : 1 und 1,25 : 1,
bevorzugt zwischen 0,9 : 1 und 1,1 : 1, liegt.

10. Verwendung von Konstruktionsmaterialien nach Ansprüchen
1 bis 8 in der medizinischen Technik, im Modellbau
oder als Schutz-, Abdeck-, Dekorations-, Abform-
oder Verglasungsmaterial.

Le A 22 537